# EUROPÄISCHE PATENTANMELDUNG

(11) **EP 3 384 785 A1**
(43) Veröffentlichungstag der Anmeldung: **10.10.2018**
(21) Anmeldenummer: 17164813.2
(22) Anmeldetag: 04.04.2017
(51) Int. Cl.: A23L 21/20, A23L 21/25, A61K 31/70, A23K 50/90, A01K 47/06, A01K 53/00

(54) **VERFAHREN ZUR HERSTELLUNG EINES HONIG ODER PROPOLIS ENTHALTENDEN LEBENSMITTELS, LEBENSMITTEL UND BIENENSTOCK**

(71) Anmelder: Gerster, Marquie-Amadeus, 88326 Aulendorf (DE)
(72) Erfinder: Gerster, Marquie-Amadeus, 88326 Aulendorf (DE)
(74) Vertreter: Otten, Roth, Dobler & Partner mbB Patentanwälte

(57) **Zusammenfassung**

1. Verfahren zur Herstellung eines Lebensmittels, insbesondere eines Honig und/oder Propolis enthaltenden Lebensmittels, umfassend folgende Verfahrensschritte:
- Bereitstellen eines Bienenstocks (1)
- Herstellen eines Futterhonigs (7) durch folgende Verfahrensschritte:
• Bereitstellen eines Ausgangsfuttermittels, welches Honig enthält, insbesondere von Honig und/oder einem Gemenge von Honig und Wachsbestandteilen
• Vermengen des Ausgangsfuttermittels mit einem Beimengungsstoff, der insbesondere teilweise feste Stoffe enthält,
• Zerkleinern der Bestandteile des Gemenges aus dem Ausgangsfuttermittel und dem Beimengungsstoff

- Verfütterung des Futterhonigs an die Bienen und/oder Zugabe des Futterhonigs zum Bienenstock (1)
- Ernten des von den Bienen des Bienenstocks (1) hergestellten Honigs und/oder des von den Bienen des Bienenstocks hergestellten Propolis.

## Beschreibung

Die Erfindung betrifft ein Verfahren zur Herstellung eines Lebensmittels, insbesondere eines Honig bzw. Propolis enthaltenden Lebensmittels, das durch dieses Verfahren hergestellte Lebensmittel sowie einen Bienenstock.

Aus dem Stand der Technik ist bekannt, dass sowohl Honig, insbesondere so genannter Manuka-Honig, als auch im Allgemeinen Propolis gesundheitsfördernde Wirkungen besitzen können. In der Regel können jedoch keine speziellen Indikationen hierfür angegeben werden, oder die Zuschreibung entsprechender Indikationen ist zuweilen umstritten, zumal es sich auch um Lebensmittel handelt und nicht um Medikamente.

Aufgabe der Erfindung ist es, ein Lebensmittel bzw. ein Verfahren zu dessen Herstellung vorschlagen zu können, bei dem gezielt gesundheitsfördernde oder spezielle Eigenschaften beeinflussbar sind.

Die Aufgabe wird, ausgehend von einem Verfahren bzw. einem Lebensmittel bzw. einem Bienenstock der eingangs genannten Art, durch die Merkmale der Ansprüche 1, 7 bzw. 8 gelöst. Durch die in den abhängigen Ansprüchen genannten Maßnahmen sind vorteilhafte Ausführungen und Weiterbildungen der Erfindung möglich.

Das erfindungsgemäße Verfahren dient zur Herstellung eines Lebensmittels, wobei es sich bei dem Lebensmittel um Honig oder Propolis handeln kann oder wobei das Lebensmittel zumindest Honig bzw. Propolis enthält. Zu dessen Herstellung wird zunächst ein Bienenstock bereitgestellt. Außerdem wird ein Futterhonig verwendet, wobei dazu ein Ausgangsfuttermaterial, das Honig enthält, bereitgestellt wird. Insbesondere kann es sich unmittelbar um Honig bzw. ein Gemenge von Honig und Wachsbestandteilen handeln. Dieses Ausgangsfuttermaterial kann später an die Bienen verfüttert werden. Zuvor jedoch wird das Ausgangsfuttermaterial mit einem Beimengungsstoff vermischt, der gegebenenfalls flüssige, zum Beispiel aber auch feste oder teilweise feste Stoffe enthalten kann. Die Bestandteile des Gemenges aus Ausgangsfuttermittel und Beimengungsstoff werden zerkleinert. Der Futterhonig wird schließlich an die Bienen verfüttert bzw. zum Bienenstock hinzugegeben. Schließlich wird das von den Bienen des Bienenstocks hergestellte Propolis oder der von diesen hergestellte Honig geerntet. Der Honig bzw. das Propolis können in ihren Eigenschaften dadurch manipuliert werden, dass entweder die dem Futterhonig zugefügten Beimengungen in dem später gewonnenen Lebensmittel enthalten sind oder dass das gewonnene Lebensmittel Stoffe, Antikörper oder dergleichen enthält, die in der Biene aufgrund der zugeführten Beimengungen erzeugt wurden. Auf diese Art und Weise kann eine neue Form des Lebensmittels gewonnen werden, welches somit gezielt verändert wird.

Die Zugabe des Beimengungsstoffs kann grundsätzlich auf verschiedene Weise zum Ausgangsfuttermittel erfolgen. Denkbar ist beispielsweise, dass das Ausgangsfuttermittel bei einem Ausführungsbeispiel der Erfindung unter Zugabe von Wasser mit dem Beimengungsstoff vermengt wird, sodass in vorteilhafter Weise auch hydrophile Bestandteile zunächst gelöst und untergemischt werden können.

Bei einem Ausführungsvarianten der Erfindung kann das Lebensmittel, welches gemäß der Erfindung hergestellt wird, so beeinflusst werden, dass dieses gezielt Stoffe enthält, die gesundheitsfördernde und sogar medizinische Wirkungen besitzen können. Beispielsweise können pflanzliche Stoffe wie zum Beispiel Algen oder Plankton als Nahrungsergänzungsmittel hinzugefügt werden. Denkbar ist auch, dass verschiedene Gräser Heublumen oder dergleichen dazu gefügt werden, um bei Allergien insbesondere eine Desensibilisierung durch regelmäßigen Konsum des gewonnen Lebensmittels durchführen zu können. Vor allem ermöglicht dieses Ausführungsbeispiel, kleinste Dosen der allergenen Substanz dem Lebensmittel zuzufügen, sodass dieses bei Einnahme über längere Zeit eine Desensibilisierungswirkung herbeiführen kann. Denkbar ist auch, dass Marihuana- oder Cannabis-haltige Bestandteile als Beimengungen verwendet werden, um zum Beispiel Schmerzpatienten mit dem Lebensmittel versorgen und deren Schmerzen lindern zu können. Auch Impfstoffe oder andere Medikamente können über das Lebensmittel zugeführt werden, indem entsprechende pharmazeutische Produkte als Beimengungsstoff verwendet werden. Schließlich ist es sogar denkbar, krankheitsauslösende Stoffe, Viren, Bakterien bzw. Organismen, die eine Krankheit verursachen können, zuzufügen, sodass im Organismus der Biene wiederum zum Beispiel Antikörper entwickelt werden, die über das gemäß der Erfindung herstellbare Lebensmittel dem Körper wiederum zuführbar sind. Dem entsprechenden Lebensmittel können gemäß einer solchen Ausführungsvariante der Erfindung somit Wirkungen eines Arzneimittels gezielt zugschrieben werden, und es kann sogar als Ersatz für entsprechende Arzneimittel verwendet werden kann.

Bei einer Ausführungsform der Erfindung wird berücksichtigt, in welcher Partikelgröße der Beimischungsstoff am besten dem Organismus der Biene zugeführt wird. Hierbei sind im Schnitt vor allem Größen zwischen 60 µm und 150 µm vorteilhaft, vorzugsweise zwischen 80 µm und 100 µm. Die Bestandteile des Gemenges aus Honig und bei Mengenanteil können also auf entsprechende Größen in vorteilhafter Weise verkleinert werden, damit die Biene diese problemlos aufnehmen kann und die Wirkung somit erhöht wird.

Bei der bevorzugten Ausführungsvariante der Erfindung kann der Futterhonig in einer Futterzarge des Bienenstocks in einen Futtertrog eingefüllt werden, und zwar insbesondere mit einer Schichtdicke von höchstens 7 mm, sodass der Rüssel der Biene diese Schicht noch hinreichend durchdringen kann. Hierdurch wird die Aufnahmemöglichkeit durch die Biene noch einmal verbessert. Ein Bienenstock kann somit in vorteilhafter Weise bei einer Weiterbildung der Erfindung als künstliche Nisthöhle für die Bienen bereitgestellt werden und einen entsprechenden Futtertrog aufweisen, sodass darin Futterhonig bevorratet werden kann, damit die Bienen daraus Futterhonig zum Verzehr entnehmen können.

Ferner kann die Futterzarge wenigstens ein lichtdurchlässiges Fenster aufweisen. Ein derartiges lichtdurchlässiges Fenster dient dazu, Sonneneinstrahlung im Bereich des Futtertrogs für die Bienen zu simulieren. Ein Bienenstock besteht in der Regel aus mehreren vertikal übereinander liegenden Bereichen, welche durch Zargen voneinander getrennt, zum Teil jedoch untereinander zugänglich sind. Im oberen Bereich des Bienenstocks kann Honig gelagert werden. Darunter befinden sich ein oder zwei Bienenräume, wobei unter anderem dort auch ein Brutraum zu finden ist. Durch den Bodenbereich können die Bienen in den Bienenstock gelangen oder heraus fliegen. Oberhalb des Honigbereichs, in dem Honig gelagert werden kann, kann nunmehr ein Futtertrog angebracht werden. Auf dem Boden des Futtertrogs wiederum kann der Futterhonig eingefüllt werden, vorzugsweise bis zu einer Schichtdicke von ca. 7 mm, damit die Biene mit ihrem Rüssel die Schicht durchdringen und diesen gut aufnehmen kann. Die entsprechende Futterzarge ist von oben zugänglich. Durch das Einfügen eines Fensters in den Futtertrog-Bereich wird die Aktivität der Bienen beeinflusst.

Die Stockbienen sind in der Regel jünger als die Flugbienen und bleiben im Stock, wo sie unter anderem mit der Pflege der Brut und der Honigverarbeitung beschäftigt sind. Der Futtertrog-Bereich wiederum wird zunächst abgesperrt, sodass die Flugbienen, die für das Einsammeln von Pollen zuständig sind, keinen Zugang zum Futtertrog haben. Die Flugbienen verlassen vor allem bei schönem Wetter bzw. Sonnenschein den Bienenstock und schwärmen aus, um z.B. von einer Wiese Pollen einzusammeln.

Sind die Flugbienen ausgeflogen, kann die Absperrung weggenommen werden, sodass das Material aus dem Futtertrog für die Stockbienen zur Verfügung steht, die den Futterhonig fressen können. Auf diese Weise wird der Futterhonig in den Verwertungskreislauf der Bienen eingepflegt, nicht jedoch wie die von den Flugbienen gesammelten Pollen an die Brut verfüttert.

Dementsprechend zeichnet sich ein erfindungsgemäßes Lebensmittel dadurch aus, dass es durch ein entsprechendes Herstellungsverfahren erhalten werden kann. Ferner zeichnet sich der erfindungsgemäße Bienenstock als künstliche Nisthöhle für Bienen und zur Durchführung des erfindungsgemäßen Herstellungsverfahrens dadurch aus, dass er einen Futtertrog umfasst, um darin Futterhonig zu bevorraten, damit für die Bienen aus dem Futtertrog Futterhonig zum Verzehr entnehmbar ist. Durch das Einfügen eines lichtdurchlässigen Fensters kann Sonnenschein simuliert werden, sodass die Bienen bemüht sind, dieses Futtermaterial in Sicherheit zu bringen.

### Ausführungsbeispiel

Ein Ausführungsbeispiel der Erfindung ist in der Zeichnung dargestellt und wird nachstehend unter Angabe weiterer Einzelheiten und Vorteile näher erläutert. Im Einzelnen zeigt:

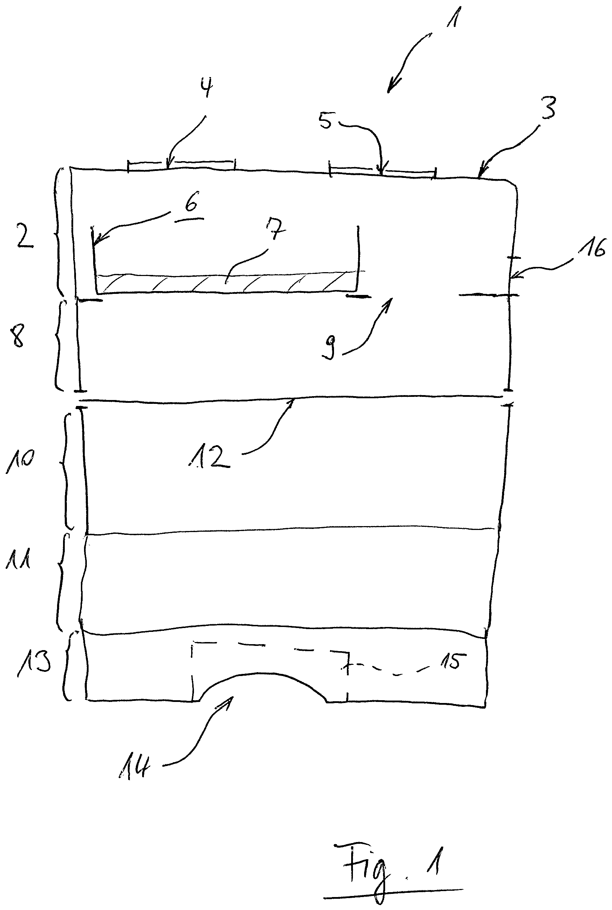

- Figur 1:: einen schematischen Schnitt durch einen Bienenstock gemäß der Erfindung.

Figur 1 zeigt ein Bienenstock 1, der aus mehreren verschiedenen Bereichen bzw. Zargen besteht. Ganz oben befindet sich die Futterzarge 2, die von einer Blende 3 mit zwei lichtdurchlässigen Fenstern 4, 5 nach oben hin abgedeckt ist. Die Fenster 4, 5 können gegebenenfalls auch abgedeckt werden, sodass kein Licht mehr hindurch gelangt. In die Futterzarge 2 wird eine Schale 6 aus lebensmittelechtem Kunststoff als Futtertrog eingebracht, in die Futterhonig eingefüllt wird und dort eine maximal 7 mm dicke Schicht 7 bildet, sodass die Schicht vom Bienenrüssel noch durchdrungen werden kann. Unter der Futterzarge 2 befindet sich ein Bereich zum Lagern von Honig, eine Honigzarge 8. Zwischen Honigzarge 8 und Futterzarge 2 befindet sich ein Durchlass 9, sodass Stock- bzw. Flugbienen hier hindurchfliegen können und von der Honigzarge 8 zur Schale 6 mit Futterhonig gelangen können.

Anschließend folgen unterhalb der Honigzarge 8 zwei Bereiche 10, 11, nämlich die sogenannten Brutzargen, in denen die Aufzucht der Brut stattfindet.

Der Bereich der Honigzarge 8 bzw. (über den Durchlass 9 zugänglich) der Futterzarge 2 ist von den Brutzargen 10, 11 durch einen Absperrboden 12 getrennt. Der Absperrboden 12 ist so im Bienenstock 1 gelagert, dass er herausgezogen werden kann, sodass im herausgezogenen Zustand ein Zugang von den Brutzargen 10, 11 zur Honigzarge 8 bzw. Futterzarge 2 möglich ist. Darunter befindet sich der Boden 13 mit einer Flugöffnung 14 zum Ein- und Ausfliegen in bzw. aus dem Bienenstock 1. Die Flugöffnung 14 kann mit einer Wabe 15 zunächst verschlossen werden, damit die Bienen sich durch diese hindurchfressen können, um so einen Eingang mit einer von den Bienen selbst gewählten Größe zu schaffen.

Bei dem Futterhonig, der in die Schale 6 eingefüllt wird, handelt es sich um Honig und/oder einem Gemenge von Honig und Wachbestandteilen, der bzw. das zusätzlich mit fein zerkleinerten Beimengungsstoffen versehen ist, insbesondere einem Medikament.

Bei schlechtem Wetter (z.B. bei Regen) fliegen die Flugbienen in der Regel nicht aus, um Pollen zu sammeln. Nun werden bei schlechtem Wetter auch über den Absperrboden 12 die Brutbereiche bzw. Brutzargen 10, 11 von der Honigzarge 8 und der Futterzarge 2 voneinander getrennt, sodass die Bienen den Futterhonig nicht an die Brut verfüttern können. Aufgrund der Fenster 4, 5 gelangt aber Licht in den Bereich der Schale 6, was die Stock- und Flugbienen, die sich im Bereich der Honigzarge 8 und der Futterzarge 2 befinden, dazu veranlasst, den Futterhonig aus der Schale 6 aufzunehmen und in den Bereich der Honigzarge 8 zu schaffen. Die Brut wird von den Stockbienen mit in den Brutzargen 10, 11 gelagerten Vorräten solange versorgt.

Bei schönem Wetter (z.B. Sonnenschein) fliegen die Flugbienen regelmäßig aus, um Pollen zu sammeln. Nun wird der Absperrboden 12 entfernt, sodass ein Durchgang zwischen Honigzarge 8 (bzw. Futterzarge 2) und Brutzargen 10, 11 wieder besteht. Das von den Flugbienen gesammelte Futter bzw. die gesammelten Pollen können an die Brut verfüttert werden. Es ist auch denkbar, in den Bereich der Honigzarge 8 oder der Futterzarge 2 einen versperrbaren Durchgang 16 als zusätzliche Öffnung für die Flugbienen vorzusehen, der bei schönem Wetter geöffnet wird.

Auf diese Weise kann der Futterhonig mitsamt den Beimengungsstoffen von den Bienen aufgenommen und weiterverarbeitet werden, sodass diese verarbeitete Masse als Honig und/oder Propolis geerntet werden kann.

### Bezugszeichen:

- 1: Bienenstock
- 2: Futterzarge
- 3: Blende
- 4: Fenster
- 5: Fenster
- 6: Schale / Futtertrog
- 7: Futterhonig-Schicht
- 8: Honigzarge
- 9: Durchlass
- 10: Brutzarge
- 11: Brutzarge
- 12: Absperrboden
- 13: Boden
- 14: Flugöffnung
- 15: Wabe
- 16: Durchgang

## Patentansprüche

**1.** Verfahren zur Herstellung eines Lebensmittels, insbesondere eines Honig und/oder Propolis enthaltenden Lebensmittels, umfassend folgende Verfahrensschritte:
- Bereitstellen eines Bienenstocks (1)
- Herstellen eines Futterhonigs (7) durch folgende Verfahrensschritte:
• Bereitstellen eines Ausgangsfuttermittels, welches Honig enthält, insbesondere von Honig und/oder einem Gemenge von Honig und Wachbestandteilen
• Vermengen des Ausgangsfuttermittels mit einem Beimengungsstoff, der insbesondere teilweise feste Stoffe enthält,
• Zerkleinern der Bestandteile des Gemenges aus dem Ausgangsfuttermittel und dem Beimengungsstoff
- Verfütterung des Futterhonigs an die Bienen und/oder Zugabe des Futterhonigs zum Bienenstock (1)
- Ernten des von den Bienen des Bienenstocks (1) hergestellten Honigs und/oder des von den Bienen des Bienenstocks hergestellten Propolis.

**2.** Verfahren zur Herstellung nach einem der vorgenannten Ansprüche, **dadurch gekennzeichnet, dass** das Ausgangsfuttermittel unter Zugabe von Wasser mit dem Beimengungsstoff vermengt wird.

**3.** Verfahren zur Herstellung nach einem der vorgenannten Ansprüche, **dadurch gekennzeichnet, dass** als Beimengungsstoff wenigstens einer der folgenden Stoffe verwendet wird:
- ein pflanzlicher Stoff, insbesondere Algen und/oder Heublumen und/oder Plankton und/oder Marihuana und/oder Cannabis-haltige Bestandteile, und/oder
- Pollen und/oder
- ein pharmazeutisches Produkt, insbesondere ein Impfstoff und/oder
- ein eine Krankheit auslösender Stoff und/oder ein Virus und/oder Organismus.

**4.** Verfahren zur Herstellung nach einem der vorgenannten Ansprüche, **dadurch gekennzeichnet, dass** die Bestandteile des Gemenges (7) aus dem Honig und dem Beimengungsstoff auf eine Größe zwischen 60 µm und 150 µm, vorzugsweise zwischen 80 µm und 100 µm, zerkleinert werden.

**5.** Verfahren zur Herstellung nach einem der vorgenannten Ansprüche, **dadurch gekennzeichnet, dass** der Futterhonig (7) in den Futtertrog (6) des Bienenstocks eingefüllt wird, insbesondere mit einer Schichtdicke von höchstens 7 mm.

**6.** Verfahren zur Herstellung nach einem der vorgenannten Ansprüche, **dadurch gekennzeichnet, dass** ein Bienenstock (1) als künstliche Nisthöhle für Bienen, umfassend einen als Futterzarge (2) ausgebildeten Bereich, um darin Futterhonig (7) zu bevorraten, damit für die Bienen aus dem Bereich der Futterzarge (2) Futterhonig zum Verzehr entnehmbar ist, verwendet wird, wobei wenigstens ein lichtdürchlässiges Fenster (4, 5) in der Futterzarge (2) vorgesehen ist, um den Futterhonig (7) mit Umgebungslicht zu bescheinen, sodass insbesondere der Futterhonig (7) von Bienen aufgenommen und weiterverarbeitet werden kann, ohne an die Bienenbrut verfüttert zu werden.

**6.** Lebensmittel, insbesondere Honig enthaltendes Lebensmittel, erhältlich durch ein Verfahren zur Herstellung nach einem der vorgenannten Ansprüche.

**7.** Bienenstock (1) als künstliche Nisthöhle für Bienen und zur Durchführung des Verfahrens zur Herstellung eines Lebensmittels, insbesondere eines Honig und/oder Propolis enthaltenden Lebensmittels nach einem der vorgenannten Ansprüche, umfassend einen als Futterzarge (2) ausgebildeten Bereich, um darin Futterhonig (7) zu bevorraten, damit für die Bienen daraus Futterhonig zum Verzehr entnehmbar ist, **dadurch gekennzeichnet, dass** die Futterzarge (2) wenigstens eine lichtdürchlässiges Fenster aufweist.

**8.** Bienenstock (1) nach einem der vorgenannten Ansprüche, **dadurch gekennzeichnet, dass** in vertikaler Richtung ein Schichtaufbau mit wenigstens folgenden Bereichen vorgesehen ist:
- ein Bodenbereich (13) als Ein- und Ausgang für die Bienen
- wenigstens ein Brutbereich (10, 11) zur Aufzucht der Brut
- eine Honigzarge (8) zum Lagern von Honig,
- eine Futterzarge (2) zum Bereitstellen von Futterhonig (2).

**9.** Bienenstock (1) nach einem der vorgenannten Ansprüche, **dadurch gekennzeichnet, dass** die Futterzarge (2) als oberster Bereich angeordnet ist und eine Absperrvorrichtung (12) zum Öffnen und Verschließen des Zugangs zwischen Burtbereich (10, 11) und Honigzarge (8) und/oder Futterzarge (2) vorgesehen ist, damit insbesondere bei schlechtem Wetter den Bienen der Zugang zum Futterhonig (7) versperrbar ist, um ein des Futterhonigs (7) Verfüttern an die Brut zu vermeiden.
